(19) Europäisches Patentamt
European Patent Office
Office européen des brevets

(11) Veröffentlichungsnummer: **0 113 317**
**B1**

(12) **EUROPÄISCHE PATENTSCHRIFT**

(45) Veröffentlichungstag der Patentschrift: **02.08.89**

(51) Int. Cl.⁴: **C 07 D 307/86**, C 07 C 125/067, C 07 C 153/00, A 01 N 47/22

(21) Anmeldenummer: **83810603.7**

(22) Anmeldetag: **19.12.83**

(54) **Carbamate.**

(30) Priorität: **24.12.82 CH 7534/82**

(43) Veröffentlichungstag der Anmeldung: **11.07.84 Patentblatt 84/28**

(45) Bekanntmachung des Hinweises auf die Patenterteilung: **02.08.89 Patentblatt 89/31**

(84) Benannte Vertragsstaaten: **BE CH DE FR GB IT LI NL**

(56) Entgegenhaltungen:
**EP-A-0 067 000**
**DE-A-1 693 155**
**DE-A-2 132 936**
**DE-A-2 142 496**
**US-A-3 539 618**

(73) Patentinhaber: **CIBA- GEIGY AG, Klybeckstrasse 141, CH- 4002 Basel (CH)**

(72) Erfinder: **Drabek, Jozef, Dr., Benkenstrasse 12, CH- 4104 Oberwil (CH)**

EP 0 113 317 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft N-Methyl-carbaminsäureester, Verfahren zu ihrer Herstellung und ihre Verwendung in der Schädlingsbekämpfung.

Die N-Methyl-carbaminsäureester haben die Formel

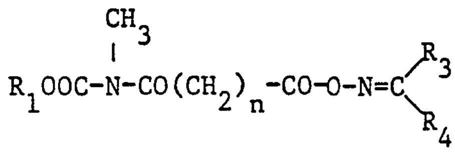 (I),

worin

R1 gegebenenfalls durch Halogen, $C_1$-$C_6$-Alkyl, $C_1$-$C_6$-Alkoxy, $C_1$-$C_6$-Alkylthio, Amino, Nitro oder Trifluormethyl substituiertes 2,3-Dihydrobenzofuranyl, Naphthyl oder Phenyl oder

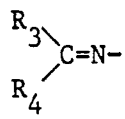 ,

$R_3$ Wasserstoff, $C_1$-$C_6$-Alkyl oder $C_2$-$C_3$-Alkenyl

$R_4$ $C_1$-$C_6$-Alkyl, $-SR_5$, 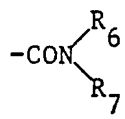 oder 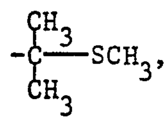,

$R_5$ $C_1$-$C_6$-Alkyl oder $C_2$-$C_3$-Alkenyl,
$R_6$ und $R_7$ je Wasserstoff oder $C_1$-$C_6$-Alkyl und
n die Zahlen 0 oder 1 bedeuten.

Unter Halogen als Substituenten am 2,3-Dihydrobenzofuranyl-, Naphthyl- und Phenylrest ist Fluor, Chlor, Brom oder Jod, insbesondere Chlor, zu verstehen.

Die bei $R_1$ und $R_3$ bis $R_7$ in Frage kommenden Alkyl-, Alkoxy-, Alkylthio- und Alkenylgruppen können geradkettig oder verzweigt sein. Beispiele von solchen Alkyl-, Alkoxy-, Alkylthio- und Alkenylgruppen sind: Methyl, Methoxy, Methylthio, Äthyl, Äthoxy, $-CH_2-CH=CH_2$, Propyl, Propoxy, Isopropyl, Isopropoxy, n-Butyl, n-Butoxy, n-Pentyl, n-Pentoxy, n-Hexyl, n-Hexoxy und deren Isomere.

Bevorzugt sind Verbindungen der Formel I,
worin

$R_1$ gegebenenfalls durch Halogen, $C_1$-$C_6$-Alkyl, $C_1$-$C_6$-Alkoxy, $C_1$-$C_6$-Alkylthio, Amino, Nitro oder Trifluormethyl substituiertes 2,3-Dihydrobenzofuranyl oder

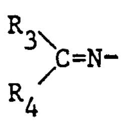 ,

$R_3$ Wasserstoff, $C_1$-$C_6$-Alkyl oder $-CH_2-CH=CH_2$,

$R_4$ $C_1$-$C_6$-Alkyl, $-SR_5$, 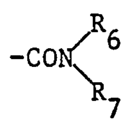 oder 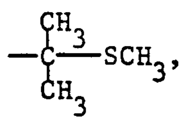,

$R_5$ $C_1$-$C_6$-Alkyl oder $-CH_2-CH=CH_2$,
$R_6$ und $R_7$ je Wasserstoff oder $C_1$-$C_6$-Alkyl und
n die Zahlen 0 oder 1 bedeuten.

Insbesondere bevorzugt sind Verbindungen der Formel I,
worin

$R_1$ gegebenenfalls durch Halogen $C_1$-$C_6$-Alkyl, $C_1$-$C_6$-Alkoxy, $C_1$-$C_6$-Alkylthio, Amino, Nitro oder Trifluormethyl substituiertes 2,3-Dihydrobenzofuranyl,
$R_3$ Wasserstoff, $C_1$-$C_6$-Alkyl oder $-CH_2-CH=CH_2$,

$R_4$ $C_1$-$C_6$-Alkyl, $-SR_5$, 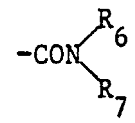 oder 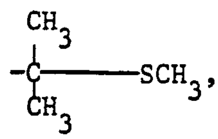

$R_5$ $C_1$-$C_6$-Alkyl oder $-CH_2-CH=CH_2$,
$R_6$ und $R_7$ je Wasserstoff oder $C_1$-$C_6$-Alkyl und
n die Zahl 0 bedeuten.

Die Verbindungen der Formel I können nach an sich bekannten Methoden z. B. wie folgt hergestellt werden:

1) 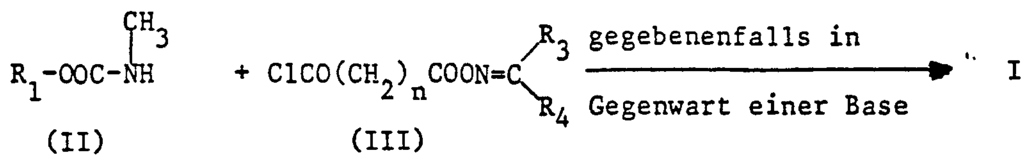

(II) (III) I

2) 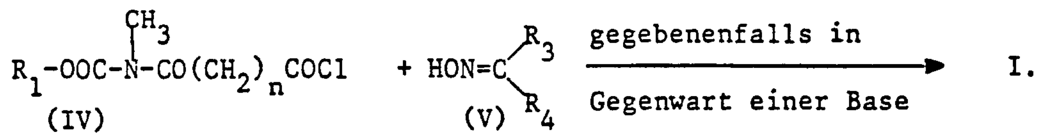

(IV) (V) I.

In den Formeln II bis V haben $R_1$, $R_3$ und $R_4$ die für die Formel I angegebene Bedeutung.

Als geeignete Basen kommen insbesondere tertiäre Amine wie Trialkylamine, Dialkylamine, p-Dialkylaminopyridine ferner Hydroxide, Oxide, Carbonate und Bicarbonate von Alkali- und Erdalkalimetallen sowie Alkalimetallalkoholate, wie z. B. Kalium-t.butylat und Natriummethylat in Betracht.

Die Verfahren werden bei normalem Druck, bei einer Temperatur von -25 bis 150° C, vorzugsweise bei -20 bis 100° C, und gegebenenfalls in einem Lösungs- oder Verdünnungsmittel durchgeführt.

Als Lösungs- oder Verdünnungsmittel eignen sich z. B. Äther und ätherartige Verbindungen wie Diäthyläther, Di-isopropyläther, Dioxan, Tetrahydrofuran; aliphatische und aromatische Kohlenwasserstoffe, insbesondere Benzol, Toluol, Xylole; Ketone wie Aceton, Methyläthylketon und Cyclohexanon.

Die Ausgangsstoffe der Formeln II bis V sind bekannt bzw. können analog bekannten Methoden hergestellt werden.

Aus der europäischen Patentveröffentlichung 0087000 sind N-Oxalyl-N-methylcarbamidsäurearylester mit insektizider und akarizider Wirkung bekannt, die aber keine Oximinogruppe aufweisen.

Die Verbindungen der Formel I eignen sich zur Bekämpfung von Schädlingen an Tieren, Pflanzen und im Boden.

Insbesondere eignen sich die Verbindungen der Formel I zur Bekämpfung von Insekten, z. B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von phytopathogenen Milben und Zecken der Ordnung Acarina. Die Verbindungen der Formel I zeigen auch gute nematozide Wirkung.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z. B. Spodoptera littoralis und Heliothis virescens) Gemüsekulturen (z. B. Leptinotarsa decemlineata und Myzus persicae), in Reis (z. B. Chilo suppressalis und Nilaparvata lugens) und von Bodeninsekten (z. B. Aulacophora femoralis, Chortophila brassicae, Pachmoda savignyi und Scotia ypsilon).

In diesem Zusammenhang ist hervorzuheben, dass die genannten Verbindungen sich sowohl durch eine stark ausgeprägte systemische als auch Kontakt-Wirkung gegen saugende Insekten und vor allem gegen Insekten der Familie Aphididae (wie z. B. Aphis fabae, Aphis craccivora und Myzus persicae), welche sich mit bekannten Mitteln nur schwierig bekämpfen lassen, auszeichnen.

Wirkstoffe der Formel I zeigen auch eine sehr günstige Wirkung gegen Fliegen, wie z. B. Musca domestica und Mückenlarven. Ferner zeichnen sie sich durch eine breite ovizide und ovolarvizide Wirkung aus und besitzen eine wertvolle Wirkung gegen ektoparasitäre Milben und Zecken z. B. der Familien Ixodidae, Argasidae und Dermanyssidae.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen

Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich Alkali-, Erdalkali- oder gegebenenfalls substituierte Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxid-Addukte an Polypropylenglykol, Äthylendiaminopropylenglykol und Alkylpolypropylenglykol mit 1 bis 10 Kohlenstoffatomen in der Alkylkette. Die genannten Verbindungem enthalten üblicherweise pro Propylenglykol-Einheit 1 bis 5 Äthylenglykol-Einheiten.

Als Beispiele nichtionischer Tenside seien Nonylphenolpolyäthoxyäthanole, Ricinusölpolyglykoläther, Polypropylen-Polyäthylenoxid-Addukte Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ridgewood, New Jersey, 1979; Dr.Helmut Stache: "Tensid Taschenbuch" Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Formulierungsbeispiele für flüssige Wirkstoffe der Formel I (% = Gewichtsprozent)**

| 1. Emulsions-Konzentrate | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyäthylenglykoläther (36 Mol ÄO) | 5 % | - | - |
| Tributylphenyl-polyäthylenglykoläther (30 Mol ÄO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80 % | 10 % | 5 % | 95 % |
| Äthylenglykol-monomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

| 3. Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

**Formulierungsbeispiele für feste Wirkstoffe der Formel I (% = Gewichtsprozent)**

| 5. Spritzpulver | a) | b) | c) |
|---|---|---|---|
| Wirkstoff | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol ÄO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff | 10 % |

| | |
|---|---|
| Octylphenolpolyäthylenglykoläther (4-5 Mol ÄO) | 3 % |
| Ca-Dodecylbenzolsulfonat | 3 % |
| Ricinusölpolyglykoläther (36 Mol ÄO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| 8. Extruder-Granulat | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 9. Umhüllungs-Granulat | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 10. Suspensions-Konzentrat | |
|---|---|
| Wirkstoff | 40 % |
| Äthylenglykol | 10 % |
| Nonylphenolpolyäthylenglykoläther (15 Mol ÄO) | 6 % |
| Na- Ligninsulfonat | 10 % |
| Carboxymethylcellulose | 1 % |
| 37 %-ige wässrige Formaldehyd-Lösung | 0,2 % |
| Silikonöl in Form einer 75 %-igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel 1:**

a) Herstellung von N-Chloroxalyl-N-methyl-7-(2,2-dimethyl-2,3-dihydrobenzofuranyl)-carbamat.

Zu einer Suspension von 22,13 g N-Methyl-7-(2,2-dimethyl-2,3-dihydrobenzofuranyl)-carbamat im 100 ml 1,2-Dichloräthan werden 25,38 g Oxalylchlorid zugetropft. Das Reaktionsgemisch wird vier Stunden bei 65°C gerührt. Nach dem Abkühlen wird das Reaktionsgemisch filtriert und aus dem Filtrat das Lösungsmittel abdestilliert. Das Rohprodukt, welches als Rückstand nach dem Abdestillieren des Lösungsmittels zurückbleibt wird aus Hexan umkristallisiert. Man erhält die Verbindung der Formel

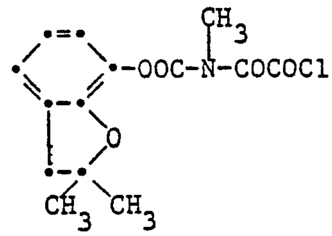

mit einem Schmelzpunkt von 99 - 101° C.

b) Herstellung von N-Äthoxycarbonyl-N-methyl-7-(2,2-dimethyl-2,3-di-hydrobenzofuranyl)-carbamat.

Zu einer Lösung von 9,35 g N-Chloroxalyl-N-methyl-7-(2,2-dimethyl-2,3-dihydrobenzofuranyl)-carbamat in 100 ml Toluol wird bei 10° C unter Rühren und Kühlen ein Gemisch von 1,52 g Äthanol und 4,56 ml Triäthylamin zugetropft. Das Gemisch wird drei Stunden bei 20° C gerührt und dann abgenutscht. Nach dem Abdestillieren des Toluols aus dem Filtrat wird der Rückstand in einem Gemisch von Hexan/Äther (1 : 1) umkristallisiert. Man erhält die Verbindung der Formel

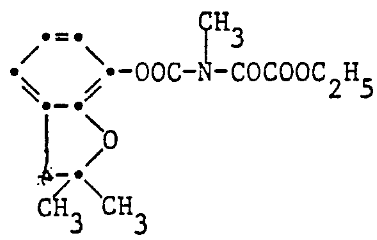

mit einem Schmelzpunkt von 82° C.

Auf analoge Weise werden auch folgende Verbindungen hergestellt:

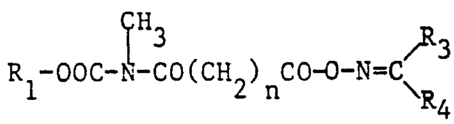

| Nr. | $R_1$ | n | $R_3$ | $R_4$ | Physikalische Daten |
|---|---|---|---|---|---|
| 1 | " | 0 | $CH_3$ | $CH_3$ | Harz |
| 2 | " | 0 | $CH_3$ | $SCH_3$ | Smp.: 127 - 128° C |
| 3 | 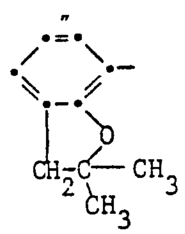 | 0 | $CH_3$ | $SC_2H_5$ | Smp.: 88 - 92° C |
| 4 | " | 0 | H | 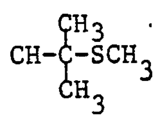 | Smp.: 90 - 95° C |

**Beispiel 2: Insektizide systemische Wirkung: Aphis craccivora**

Bewurzelte Bohnenpflanzen werden in Töpfe, welche 600 ccm Erde enthalten, verpflanzt. Anschliessend werden 50 ml einer Versuchslösung, enthaltend 25 ppm, 5 ppm bzw. 1 ppm der zu prüfenden Verbindung, direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 48 Stunden nach Versuchsbeginn. Pro Konzentrationsdosis Testsubstanz werden zwei Pflanzen, je eine in einem separaten Topf, verwendet. Der Versuch wird bei 25° C und 70 % relativer Luftfeuchtigkeit durchgeführt.

Die Verbindungen Nr. 1 - 4 haben bei 1 ppm eine 70 - 100 % Wirkung gegen Aphis craccivora.

## Patentansprüche

1. Ein N-Methyl-carbaminsäureester der Formel

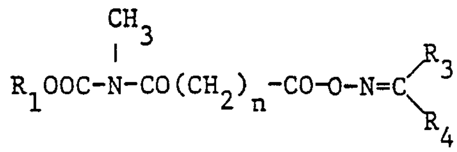

(I),

worin

$R_1$ gegebenenfalls durch Halogen, $C_1$-$C_6$-Alkyl, $C_1$-$C_6$-Alkoxy, $C_1$-$C_6$-Alkylthio, Amino, Nitro oder Trifluormethyl substituiertes 2,3-Dihydrobenzofuranyl, Naphthyl oder Phenyl oder

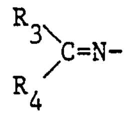

,

$R_3$ Wasserstoff, $C_1$-$C_6$-Alkyl oder $C_2$-$C_3$-Alkenyl,

$R_4$ $C_1$-$C_6$-Alkyl, $-SR_5$, 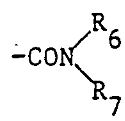 oder 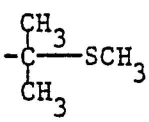,

$R_5$ $C_1$-$C_6$-Alkyl oder $C_2$-$C_3$-Alkenyl,
$R_6$ und $R_7$ je Wasserstoff oder $C_1$-$C_6$-Alkyl und
n die Zahlen 0 oder 1 bedeuten.

2. Eine Verbindung gemäss Anspruch 1, worin

$R_1$ gegebenenfalls durch Halogen, $C_1$-$C_6$-Alkyl, $C_1$-$C_6$-Alkoxy, $C_1$-$C_6$-Alkylthio, Amino, Nitro oder Trifluormethyl substituiertes 2,3-Dihydrobenzofuranyl oder

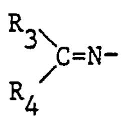,

$R_3$ Wasserstoff, $C_1$-$C_6$-Alkyl oder $-CH_2-CH=CH_2$,

$R_4$ $C_1$-$C_6$-Alkyl, $-SR_5$, 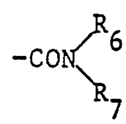 oder 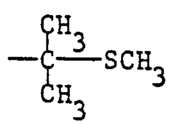,

$R_5$ $C_1$-$C_6$-Alkyl oder $-CH_2-CH=CH_2$,
$R_6$ und $R_7$ je Wasserstoff oder $C_1$-$C_6$-Alkyl und
n die Zahlen 0 oder 1 bedeuten.

3. Eine Verbindung gemäss Anspruch 2, worin

$R_1$ gegebenenfalls durch Halogen, $C_1$-$C_6$-Alkyl, $C_1$-$C_6$-Alkoxy, $C_1$-$C_6$-Alkylthio, Amino, Nitro oder Trifluormethyl substituiertes 2,3-Dihydrobenzofuranyl
$R_3$ Wasserstoff, $C_1$-$C_6$-Alkyl oder $-CH_2-CH=CH_2$,

$R_4$ $C_1$-$C_6$-Alkyl, $-SR_5$, 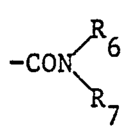 oder 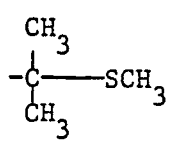,

$R_5$ $C_1$-$C_6$-Alkyl oder $-CH_2-CH=CH_2$,
$R_6$ und $R_7$ je Wasserstoff oder $C_1$-$C_6$-Alkyl und
n die Zahlen 0 oder 1 bedeuten.

4. Die Verbindung gemäss Anspruch 3 der Formel

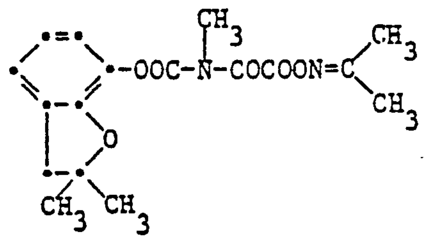

5. Die Verbindung gemäss Anspruch 3 der Formel

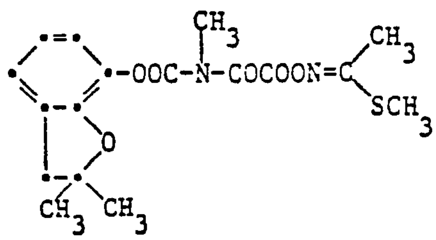

6. Die Verbindung gemäss Anspruch 3 der Formel

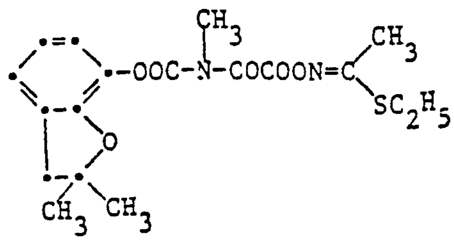

7. Die Verbindung gemäss Anspruch 3 der Formel

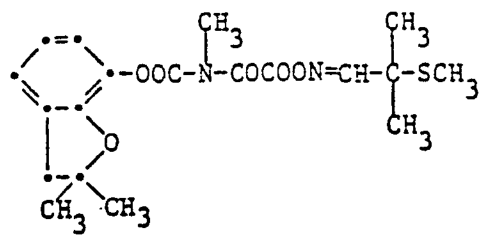

8. Ein Verfahren zur Herstellung von Verbindungen gemäss Anspruch 1, dadurch gekennzeichnet, dass man gegebenenfalls in Gegenwart einer Base eine Verbindung der Formel

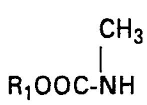

mit einer Verbindung der Formel

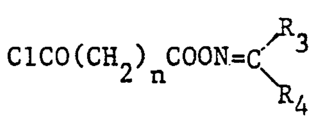

umsetzt, worin $R_1$, $R_3$, $R_4$ und n die im Anspruch 1 angegebene Bedeutung haben.

9. Ein Schädlingsbekämpfungsmittel, welches neben geeigneten festen oder flüssigen Zusatzstoffen als aktive Komponente eine Verbindung gemäss Anspruch 1 enthält.

10. Die Verwendung einer Verbindung der Formel I gemäss Anspruch 1 zur Bekämpfung von Schädlingen an Tieren und Pflanzen sowie im Boden, mit der Massgabe, dass die Wirksubstanz nicht zur therapeutischen Behandlung von Warmblütern eingesetzt wird.

11. Die Verwendung gemäss Anspruch 10 zur Bekämpfung von Insekten und Vertretern der Ordnung Akarina.

## Claims

1. An N-methyl-carbamic acid ester of the formula

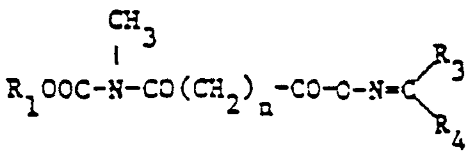 (I)

wherein

$R_1$ is 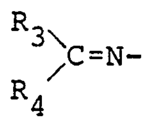

or is 2,3-dihydrobenzofuranyl, naphthyl or phenyl, each unsubstituted or substituted by halogen, $C_1$-$C_6$-alkyl, $C_1$-$C_6$-alkoxy, $C_1$-$C_6$-alkylthio, amino, nitro or trifluoromethyl,
$R_3$ is hydrogen, $C_1$-$C_6$-alkyl or $C_2$-$C_3$-alkenyl,
$R_4$ is $C_1$-$C_6$-alkyl, $-SR_5$, 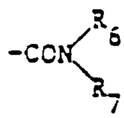 or 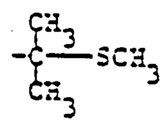 ,
$R_5$ is $C_1$-$C_6$-alkyl or $C_2$-$C_3$-alkenyl,
$R_6$ and $R_7$ are each hydrogen or $C_1$-$C_6$-alkyl, and
n is zero or 1.

2. A compound according to claim 1, wherein

$R_1$ is 2,3-dihydrobenzofuranyl or

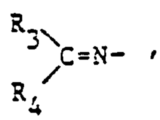 ,

each unsubstituted or substituted by halogen, $C_1$-$C_6$-alkyl, $C_1$-$C_6$-alkoxy, $C_1$-$C_6$-alkylthio, amino, nitro or trifluoromethyl,
$R_3$ is hydrogen, $C_1$-$C_6$-alkyl or $-CH_2-CH=CH_2$,
$R_4$ is $C_1$-$C_6$-alkyl, $-SR_5$, 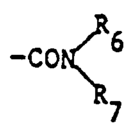 or 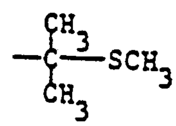 ,
$R_5$ is $C_1$-$C_6$-alkyl or $-CH_2-CH=CH_2$,
$R_6$ and $R_7$ are each hydrogen or $C_1$-$C_6$-alkyl, and
n is zero or 1.

3. A compound according to claim 2, wherein

$R_1$ is 2,3-dihydrobenzofuranyl which is unsubstituted or substituted by halogen, $C_1$-$C_6$-alkyl, $C_1$-$C_6$-alkoxy, $C_1$-$C_6$-alkylthio, amino, nitro or trifluoromethyl,
$R_3$ is hydrogen, $C_1$-$C_6$-alkyl or $-CH_2-CH=CH_2$,
$R_4$ is $C_1$-$C_6$-alkyl, $-SR_5$, 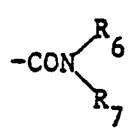 or 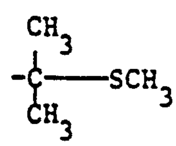 ,
$R_5$ is $C_1$-$C_6$-alkyl or $-CH_2-CH=CH_2$,
$R_6$ and $R_7$ are each hydrogen or $C_1$-$C_6$-alkyl, and
n is zero.

4. The compound according to claim 3 of the formula

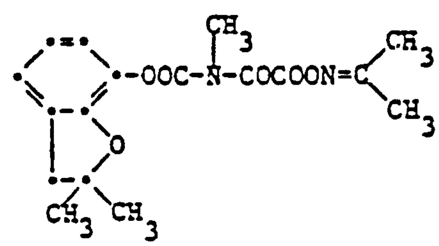

5. The compound according to claim 3 of the formula

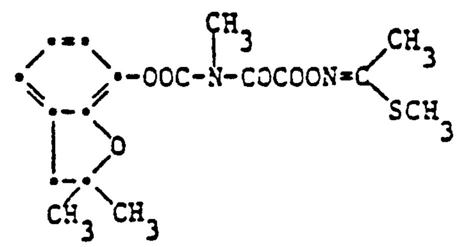

6. The compound according to claim 3 of the formula

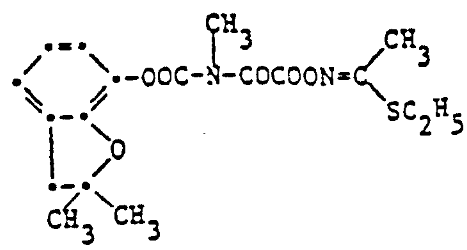

7. The compound according to claim 3 of the formula

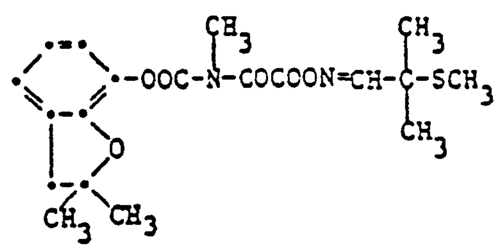

8. A process for producing compounds according to claim 1, which process comprises reacting a compound of the formula

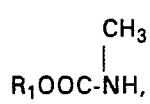

optionally in the presence of a base, with a compound of the formula

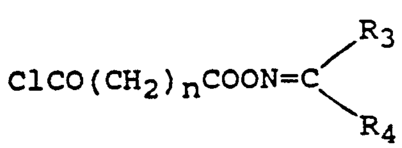

wherein $R_1$, $R_3$, $R_4$ and n have the meanings defined in claim 1.

9. A pesticidal composition which contains as active ingredient a compound according to claim 1, together with suitable solid or liquid additives.

10. The use of a compound of formula I according to claim 1 for controlling pests on animals and plants as well as in the soil, with the proviso that the active ingredient is not used for the therapeutic treatment of warm-blooded animals.

11. The use according to claim 10 for controlling insects and representatives of the order Acarina.

## Revendications

1. Un ester N-méthylcarbamique de formule

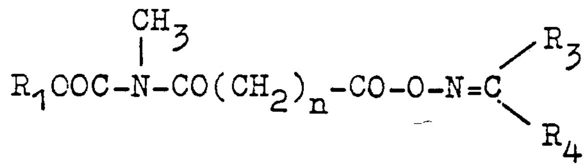

(I),

dans laquelle

$R_1$ représente un groupe 2,3-dihydrobenzofurannyle, naphtyle ou phényle éventuellement substitué par des halogènes, des groupes alkyle en $C_1$-$C_6$, alcoxy en $C_1$-$C_6$, alkylthio en $C_1$-$C_6$, amino, nitro ou trifluorométhyle, ou le groupe

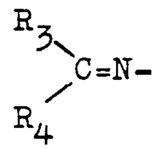

,

$R_3$ représente l'hydrogène, un groupe alkyle en $C_1$-$C_6$ ou alcényle en $C_2$-$C_6$,
$R_4$ représente un groupe alkyle en $C_1$-$C_6$, $-SR_5$,

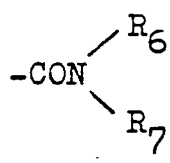

ou

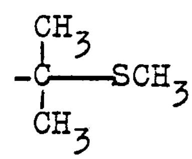

,

$R_5$ représente un groupe alkyle en $C_1$-$C_6$ ou alcényle en $C_2$-$C_3$,
$R_6$ et $R_7$ représentent chacun l'hydrogène ou un groupe alkyle en $C_1$-$C_6$, et
n est égal à 0 ou 1.

2. Un composé selon la revendication 1, dans lequel

$R_1$ représente un groupe 2,3-dihydrobenzofurannyle éventuellement substitué par des halogènes, des groupes alkyle en $C_1$-$C_6$, alcoxy en $C_1$-$C_6$, alkylthio en $C_1$-$C_6$ amino, nitro ou trifluorométhyle, ou le groupe

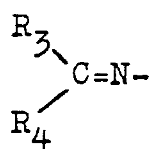

,

$R_3$ représente l'hydrogène, un groupe alkyle en $C_1$-$C_6$ ou $-CH_2-CH=CH_2$,
$R_4$ représente un groupe alkyle en $C_1$-$C_6$, $-SR_5$,

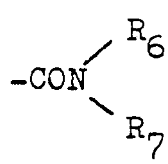

ou

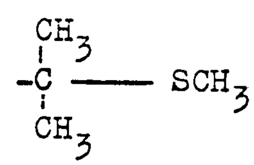

,

$R_5$ représente un groupe alkyle en $C_1$-$C_6$ ou $-CH_2-CH=CH_2$,
$R_6$ et $R_7$ représentent chacun l'hydrogène ou un groupe alkyle en $C_1$-$C_6$, et
n est égal à 0 ou 1.

3. Un composé selon la revendication 2, dans lequel

$R_1$ représente un groupe 2,3-dihydrobenzofurannyle éventuellement substitué par des halogènes, des groupes alkyle en $C_1$-$C_6$, alcoxy en $C_1$-$C_6$, alkylthio en $C_2$-$C_6$, amino, nitro ou trifluorométhyle,

$R_3$ représente l'hydrogène, un groupe alkyle en $C_1$-$C_6$ ou $-CH_2-CH=CH_2$,
$R_4$ représente un groupe alkyle en $C_1$-$C_6$, $-SR_5$,

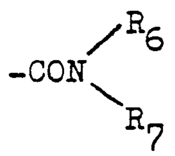
ou
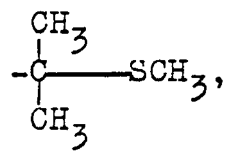
,

$R_5$ représente un groupe alkyle en $C_1$-$C_6$ ou $-CH_2-CH=CH_2$,
$R_6$ et $R_7$ représentent chacun l'hydrogène ou un groupe alkyle en $C_1$-$C_6$, et
n est égal à 0.

4. Le composé selon la revendication 3, de formule

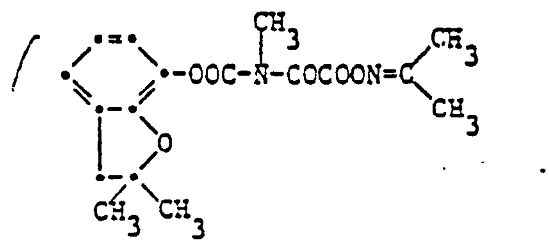

5. Le composé selon la revendication 3, de formule

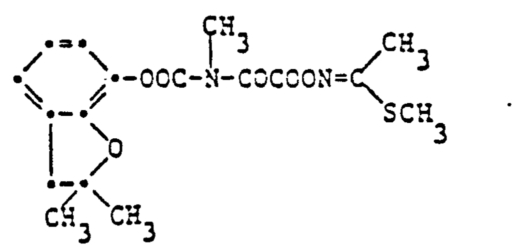

6. Le composé selon la revendication 3, de formule

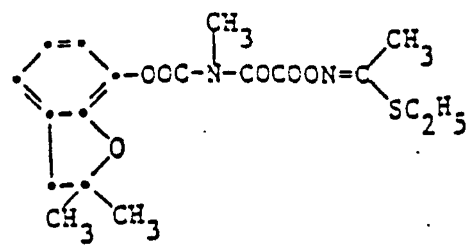

7. Le composé selon la revendication 3, de formule

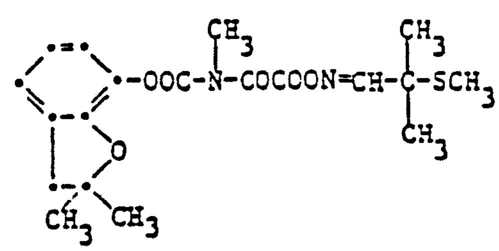

8. Un procédé de préparation des composés selon la revendication 1, caractérisé en ce que l'on fait réagir, éventuellement en présence d'une base, un composé de formule

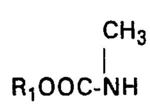

avec un composé de formule

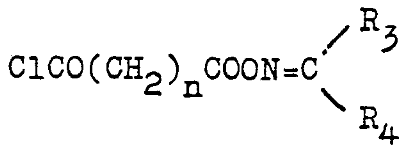

dans lesquelles $R_1$, $R_3$, $R_4$ et n ont les significations indiquées dans la revendication 1.

9. Un produit pesticide contenant en tant que composant actif, avec des additifs solides ou liquides appropriés, un composé selon la revendication 1.

10. L'utilisation d'un composé de formule I selon la revendication 1 dans la lutte contre les parasites des animaux et des végétaux ou du sol, sous réserve que la substance active ne peut être utilisée pour le traitement thérapeutique des individus à sang chaud.

11. L'utilisation selon la revendication 10, dans la lutte contre les insectes et les représentants de l'ordre des acariens.